# EUROPÄISCHE PATENTSCHRIFT

(11) **EP 0 300 287 B1**
(45) Veröffentlichungstag und Bekanntmachung des Hinweises auf die Patenterteilung: **07.01.1993**
(21) Anmeldenummer: 88110912.8
(22) Anmeldetag: 08.07.1988
(51) Int. Cl.: C12P 13/00, C12P 7/62, C07C 227/16, C07C 29/136

(54) **Verfahren zur Reduktion von Ketonen**
Process for the reduction of ketones
Procédé de réduction de cétones

(30) Priorität: 22.07.1987 DE 3724197
(43) Veröffentlichungstag der Anmeldung: 25.01.1989
(73) Patentinhaber: MERCK PATENT GmbH, D-64271 Darmstadt (DE)
(72) Erfinder: Radunz, Hans-Eckart, Dr., D-6109 Mühltal (DE); Schwartz, Harry, Dr., D-6203 Hochheim/Main (DE); Heinrich, Martin, Dr., D-6100 Darmstadt (DE)

(56) Entgegenhaltungen:
- EP-A- 0 077 028
- EP-A- 0 104 041
- EP-A- 0 228 192
- US-A- 4 477 440
- TETRAHEDRON LETTERS, vol. 27, Nr. 23, 1986 (Oxford, GB), K. USHIO et al. "Stereochemical Control in Microbial Reduction 4. Effect of Cultivation Conditions on the Reduction of b-Keto Esters by Methylotrophic Yeasts, S. 2657-2660
- THE JOURNAL OF ORGANIC CHEMISTRY, VOL. 52, Nr. 2, 23. Jänner 1987, (Washington DC), D.W. BROOKS et al. "Substrate Modification as a Means of Enhancing the Enantioselectivity of Microbial Reductions of b-Keto Esters: An (R)-or (S)-1,3,5-Trihydrosypentane Synthon", S. 192-196

## Beschreibung

Die Erfindung betrifft ein mikrobielles Verfahren zur enantioselektiven Reduktion von Ketoverbindungen der Formel I

R³HN-CHR¹-CO-CH₂-COOR² I,

worin
- R¹: A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen oder Bicycloalkyl mit 7-14 C-Atomen oder Bicycloalkylalkyl mit 8-18 C-Atomen,
- R²: H oder Alkyl mit 1-5 C-Atomen
- R³: H, Benzyl, Triphenylmethyl, Benzyloxycarbonyl, tert.-Butyloxycarbonyl oder 9-Fluorenylmethoxycarbonyl,
- Ar: unsubstituiertes oder ein- oder mehrfach durch A, AO, Hal, CF₃, HO, Hydroxyalkyl mit 1-8 C-Atomen, H₂N und/oder Aminoalkyl mit 1-8 C-Atomen substituiertes Phenyl oder unsubstituiertes Naphthyl,
- Het: einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, CF₃, HO, O₂N, Carbonylsauerstoff, H₂N, HAN, A₂N, AcNH, AS, ASO, ASO₂, AOOC, CN, H₂NCO, HOOC, H₂NSO₂, ASO₂NH, Ar, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,
- Hal: F, Cl, Br oder J,
- Ac: A-CO-, Ar-CO- oder A-NH-CO- und
- A: Alkyl mit 1-8 C-Atomen bedeuten,
bedeuten,
zu den entsprechenden sekundären Alkoholen, die wichtige Zwischenstufen bei der Synthese von pharmakologisch wertvollen Renin-Inhibitoren darstellen.

Verbindungen der Formel II

R³NH-CHR¹-CHOH-CH₂-COOR² II,

worin R¹ bis R³ die angegebenen Bedeutungen haben, sind Derivate der natürlich vorkommenden γ-Aminosäure Statin. Um für die Renin-Inhibierung wirksam zu sein, muß die OH-Gruppe am β-C-Atom der Verbindungen der Formel II S-konfiguriert sein ebenso wie die Aminogruppe am γ-C-Atom. Zur Herstellung der sekundären Alkohole eignen sich bevorzugt die entsprechenden Ketoverbindungen der Formel I. Die Reduktion dieser Ketoverbindungen nach bekannten Standardmethoden, beispielsweise mit LiAlH₄, NaBH₄, Na/NH₃ oder Li/NH₃, gelingt aufgrund der erleichterten Enolisierbarkeit des Sauerstoffs am β-C-Atom in nur sehr schlechten Ausbeuten. Reduktion mit Raney-Nickel bewirkt einen Ausbeuteanstieg (Steulmann u. Klostermeyer, Liebigs Ann.Chem., S. 2245-2250 (1975)). Allen diesen Reduktionen derartiger β-Ketoverbindungen ist jedoch gemeinsam, daß ein Diastereomerengemisch aus R- und S-Form des betreffenden sekundären Alkohols meist zu gleichen Teilen gebildet wird. Dies bedeutet, daß nach abgeschlossener Reduktion in der Regel sich eine zumeist aufwendige Diastereomerentrennung anschließen muß. Das führt wiederum zu oft erheblichen Ausbeuteverlusten.

In jüngerer Zeit werden zur Reduktion von Ketoverbindungen, insbesondere β-Ketoestern, zuweilen Hefen eingesetzt, welche weitgehend enantioselektiv zu reduzieren vermögen (z.B. Seebach et al., Org.Synth. 63, 1-9 (1985); Ushio et al., Tetrahedron Lett. Vol. 27, No. 23, 2657-2660 (1986); Brooks et al., J.Org.Chem. 52, 192-196 (1987)). In diesen Systemen ist aber oft ein starker Einfluß der in Nachbarschaft zum Reaktionszentrum stehenden Gruppen erkennbar: geringe Änderungen können zu erheblich schlechteren Ausbeuten und ungünstigen Enantiomerenverhältnissen führen, so daß sich mit Hilfe von Hefen bislang nur eine beschränkte Palette recht spezieller β-Ketoverbindungen zufriedenstellend enantioselektiv reduzieren ließ. Oftmals erhält man auch das "falsche" Enantiomer.

Es bestand also die Aufgabe, ein Verfahren zu finden, das die Herstellung von wertvollen Verbindungen der Formel II in hohen Ausbeuten stereospezifisch und auf einfache und wirtschaftliche Weise ermöglicht.

Überraschend wurde gefunden, daß sich die β-Ketoverbindungen der Formel I mit Hilfe von Mikroorganismen, insbesondere Hefen, in guten Ausbeuten zu den am β-C-Atom S-konfigurierten, biologisch aktiven, sekundären Alkoholen der Formel II reduzieren lassen. Dies war nach dem bisherigen Kenntnisstand nicht voraussehbar, insbesondere in Anbetracht der besonderen und teilweise reaktiven Gruppen, die die Ausgangsverbindungen enthalten.

Gegenstand der Erfindung ist somit ein Verfahren zur enantioselektiven Reduktion von Ketoverbindungen zu sekundären Alkoholen mit Hilfe von Mikroorganismen, dadurch gekennzeichnet, daß man Verbindungen der Formel I einsetzt.

Gegenstand der Erfindung ist ferner die Verwendung der Verbindungen der Formel II, hergestellt durch Reduktion von Verbindungen der Formel I mit Hilfe von Mikroorganismen, in der Synthese von Renin-Inhibitoren, die das Strukturelement -NH- 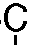 H-CHOH-CH₂-CO- enthalten.

Vor- und nachstehend haben die Reste R¹, R², R³, Ar, Het, Hal, Ac und A die angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl. Substituiertes Cycloalkyl bedeutet vorzugsweise 1-, 2- oder 3-Methylcyclopentyl, 1-, 2-, 3- oder 4-Methylcyclohexyl, 4-tert.-Butylcyclohexyl, wobei die Substituenten vorzugsweise in trans-Stellung zueinander angeordnet sind.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2- oder 3-Methylcyclopentylmethyl, 1-, 2-, 3-, 4-Methylcyclohexylmethyl oder 4-tert.-Butylcyclohexylmethyl, wobei die Substituenten vorzugsweise in trans-Stellung zueinander angeordnet sind.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Hal bedeutet vorzugsweise F, Cl oder Br, aber auch J.

Ac bedeutet vorzugsweise A-CO- wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m- oder p-Hydroxyphenyl, o-, m- oder p-Hydroxymethylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m- oder p-Aminomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m- oder p-Hydroxybenzyl, o-, m- oder p-Hydroxymethylbenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, 1- oder 2-Naphthylmethyl.

Het ist vorzugsweise 2- oder 3-Furyl, 2- oder 3-Thienyl, 1-, 2- oder 3-Pyrryl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6 oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6-, 7- oder 8-Chinazolyl. Die heterocyclischen Reste können auch teilweise oder vollständig hydriert sein. Het kann also z. B. auch bedeuten 2,3-Dihydro-2-, -3-, -4- oder -5-furyl, 2,5-Dihydro-2-, -3-, -4- oder-5-furyl, Tetrahydro-2- oder -3-furyl, Tetrahydro-2- oder -3-thienyl, 2,3-Dihydro-1-, -2-, -3-, -4- oder -5-pyrryl, 2,5-Dihydro-1-, -2-, -3-, -4- oder -5-pyrryl, 1-, 2- oder 3-Pyrrolidinyl, Tetrahydro-1-, -2- oder -4-imidazolyl, 2,3-Dihydro-1-, -2-, -3-, -4- oder -5-pyrazolyl, 2,5-Dihydro-1-, -2-, -3-, -4- oder -5-pyrazolyl, Tetrahydro-1-, -3-, oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrryl, 1-Methyl-4- oder -5-nitro-2-pyrryl, 3,5-Dimethyl-4-ethyl-2-pyrryl, 4-Methyl-5-pyrazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N′-Methylureido-1H-4-pyridon-5-yl, 5- oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl.
- R¹: bedeutet vorzugsweise Benzyl, Cyclohexylmethyl, 4-Methylcyclohexylmethyl, 4-tert.-Butylcyclohexylmethyl, aber auch Phenyl, p-Chlorbenzyl, 2-Phenylethyl, 2-Cyclohexylethyl, 1- oder 2-Naphthylmethyl sowie ferner Methyl, Ethyl, Propyl, Isopropyl, Butyl, Isobutyl, sek.-Butyl, Pentyl oder 2- oder 3-Methylbutyl.
- R²: bedeutet vorzugsweise Alkyl mit 1-3 C-Atomen, also Methyl, Ethyl, Propyl oder Isopropyl
- R³: bedeutet vorzugsweise H, Benzyloxycarbonyl oder tert.-Butyloxycarbonyl.

Die als Ausgangsverbindungen dienenden Ketoverbindungen der Formel I (im folgenden"Ketostatine") sind entweder bekannt oder lassen sich in an sich bekannter Weise nach Standardmethoden der organischen Chemie herstellen. Vorzugsweise werden α-Aminosäureester der Formel IV

R³HN-CHR¹-COOR⁴ IV,

worin R¹ und R³ die angegebenen Bedeutungen haben und R⁴ eine leicht abspaltbare Carboxy-Schutzgruppe, z.B. Methyl, Ethyl, Benzyl oder Phenacyl darstellt, mit Malonsäurederivaten der Formel V

R⁵OOC-CH₂-COOR² V,

worin R² die angegebene Bedeutung hat, und
- R⁵: H, Alkyl oder MgBr⁺ bedeutet,
kondensiert, hydrolysiert und decarboxyliert. Die Versuchsbedingungen entsprechen denen der üblichen Malonestersynthesen. Eine vergleichbare Synthese von 4-Amino-3-oxo-6-methylheptansäureethylester ist bei Steulmann u. Klostermeyer, l.c., ausführlich beschrieben.

Falls R³ nicht H ist, hat es die Aufgabe einer wieder abspaltbaren Aminoschutzgruppe. Bei der erfindungsgemäßen Reduktion der Ketostatine mittels Mikroorganismen ist die Anwesenheit einer Aminoschutzgruppe - meist im Gegensatz zur klassischen chemischen Reduktion - nicht erforderlich. Für bestimmte Aufarbeitungstechniken im Anschluß an die Reduktion, insbesondere dann, wenn das Reaktionsprodukt in organische Phasen übergeführt werden soll, ist hingegen das Vorhandensein einer Aminoschutzgruppe sehr nützlich, da das nicht geschützte Produkt in der Regel wasserlöslich ist. Falls die Reduktion des Ketostatins mit einer Aminoschutzgruppe durchgeführt wird, darf diese keine durch Mikroorganismen reduzierbare Ketogruppe enthalten.

Die Einführung der Aminoschutzgruppe in die Ketostatine entspricht der literaturbekannten Standardmethodik. Vorzugsweise werden hierfür tert.-Butyloxycarbonyl (BOC) oder Benzyloxycarbonyl (Z) eingesetzt.

Als Mikroorganismen eignen sich prinzipiell alle solche Spezies, welche wasserstoffübertragende Enzyme (Dehydrogenasen) enthalten. Besonders bevorzugt sind Hefen, da sie einerseits relativ hohe Dehydrogenaseaktivitäten aufweisen, andererseits leicht verfügbar sind und nur einfache Züchtungsbedingungen erfordern.

Unter den Hefen eignen sich vorzugsweise die Gattungen Saccharomyces, Candida, Hansenula, Pullularia, Torulopsis oder Williopsis. Besonders bevorzugt sind Hefen der Gattung Hansenula, insbesondere die Arten Hansenula ciferrii, Hansenula saturnus oder Hansenula anomala. Die bevorzugten Hefen sind entweder käuflich erwerbbar oder über eine hierfür autorisierte Hinterlegungsstelle allgemein zugänglich (z.B. Hansenula ciferrii: DSM 70780; Hansenula saturnus: DSM 70278; Saccharomyces uvarum: DSM 70547; Torulopsis candida: DSM 70590 oder Hansenula anomala: DSM 70130).

Die Züchtung der Mikroorganismen und die Gewinnung von Zellmasse zu Versuchszwecken erfolgt in hierfür geeigneten, allgemein gängigen Medien nach bekannter Methodik. Die nach dem erfindungsgemäßen Verfahren bevorzugten Hefen, insbesondere der Gattung Hansenula, werden vorzugsweise auf einem Yeast-Extract-Pepton-Medium (YEP) mit beispielsweise folgender Zusammensetzung: Pepton (2 %), Glucose (2 %), KH₂PO₄ (0,1 %), Agar (2 %), Hefeextrakt (1 %); pH-Wert: 6,5 gehalten. Die Gewinnung von Zellmasse erfolgt vorzugsweise in einem Medium bestehend aus 0,3 % Hefeextrakt, 0,3 % Malzextrakt, 2 % Glucose und 0,5 % Pepton (pH = 6,5). Die Mengenangaben sind nur beispielhaft. Hieraus wird eine 20-30 h alte Kultur in bekannter Weise gezogen. Nach Abschluß der Kultur werden die Zellen vorzugsweise abzentrifugiert und bis zur Umsetzung tiefgefroren.

Nach dem erfindungsgemäßen Verfahren werden als Ketostatine vorzugsweise Verbindungen der Formel I eingesetzt, bei denen R¹ Benzyl, 4-Methyl-cyclohexylmethyl oder 4-tert.-Butyl-cyclohexylmethyl, R² Methyl oder Ethyl und R³ H oder eine Aminoschutzgruppe, wie oben angegeben, bedeutet.

Vorzugsweise wird das erfindungsgemäße Verfahren wie folgt durchgeführt. Das betreffende Ketostatin wird, insbesondere wenn es eine Aminoschutzgruppe enthält, in Ethanol gelöst, wobei die Ethanolkonzentration zwischen 3-10 %, und die Ketostatinkonzentration 0,05-0,5 %, vorzugsweise 0,1-0,2 %, bezogen auf das Inkubationsvolumen, beträgt. Die ethanolische Ketostatinlösung wird zu einer wäßrigen 5-30 %igen, vorzugsweise 10-20 %igen, Glucoselösung, in der zuvor die tiefgefrorenen Hefezellen suspendiert wurden, gegeben. Die Hefekonzentration variiert zwischen 0,5 und 5 %, vorzugsweise aber zwischen 1 und 2 %. Die Reduktion erfolgt unter ständigem Rühren zwischen 25 und 30 °C, vorzugsweise bei 28 °C, unter Gärbedingungen oder unter leichter Belüftung. Letztere ist insbesondere bei Durchführung des erfindungsgemäßen Verfahrens im technischen Maßstab bevorzugt. Die Inkubationsdauer liegt zwischen 24 und 84 Stunden, vorzugsweise zwischen 48 und 72 Stunden.

In einer weiteren Ausführungsform des erfinderischen Verfahrens kann die Reduktion, statt - wie geschildert - mit ruhenden auch mit wachsenden Zellen durchgeführt werden, wodurch sich vorteilhafterweise die Vorkultivierung erübrigt. In möglichst wenig Ethanol gelöstes Ketostatin wird einer Zellensuspension in einem literaturüblichen Wachstumsmedium, bestehend beispielsweise aus Glucose (5 %), m-Inosit (0,004 %), KH₂PO₄ (0,1 %), MgSO₄ (0,1 %), Kaliumhydrogentartrat (0,45 %), Spurenelementen und Vitaminen (pH 5-6), zugesetzt und die Reduktion unter gleichen Bedingungen, wie oben für ruhende Zellen angegeben, durchgeführt.

Die Aufarbeitung, Reinigung und Analyse des gebildeten sekundären Alkohols ist für ruhende und wachsende Zellen identisch. Enthält das Edukt/Produkt eine Aminoschutzgruppe, wird die Zellsuspension einige Male mit einem organischen Lösungsmittel, beispielsweise Ethylacetat, Dichlormethan, Diethylether oder Butylmethylether ausgeschüttelt. Gegebenenfalls kann sich eine weitere Reinigung beispielsweise mit Kieselgelin in an sich bekannter Weise anschließen. Da erfindungsgemäß ein hoher Überschuß eines Enantiomeren gebildet wird, kann eine Aufreinigung auch durch selektive Kristallisation nach bekannter Methodik bewerkstelligt werden. Hierzu wird die organische Phase bis zum Rückstand eingeengt und in einem zur Aus-/Umkristallisation geeigneten organischen Lösungsmittel, gelöst und auskristallisiert. Nachweis und Bestimmung der Enantiomerenverhältnisses (R- und S-Form) läßt sich in an sich bekannter Weise z.B. durch Dünnschichtchromatographie oder HPLC durchführen. Aus dem Endprodukt kann bei Bedarf in an sich bekannter Weise die Aminoschutzgruppe abgespalten werden.

Enthält das betreffende Ketostatin keine Aminoschutzgruppe (R³ = H), so ist eine Extraktion mit organischen Lösungsmitteln nach abgeschlossener Reduktion in der Regel nicht möglich, da der gebildete Alkohol in seiner nicht aminogeschützten Form wasserlöslich ist und eine vorhergehende Alkalisierung zur Cyclisierung führt. Der betreffende Alkohol kann aber direkt aus der Reaktionssuspension nach Abtrennung der Zellen und anschließender Gefriertrocknung gewonnen werden. Aufreinigung und Nachweis entsprechen der geschützten Form.

In einer weiteren Ausführungsform der erfindungsgemäßen Verfahren kann die Reduktion des Ketostatins mit immobilisierten Zellen durchgeführt werden. Hierbei werden die Zellen in bekannter Weise in eine übliche Trägermatrix, z.B. ein Polyacrylamidgel eingebracht. Die Trägermatrix wird zerkleinert und anstelle "freier" Zellen zur Reduktion unter sonst unveränderten, oben geschilderten Bedingungen eingesetzt. Dabei kann die Reaktion im Batch-Verfahren oder im Säulenbetrieb durchgeführt werden. Auch eine Mehrfachverwendung des mit Zellen beladenen Gels ist möglich, wobei allerdings die Reduktionsleistung der immobilisierten Zellen etwas abnimmt. Die Extraktion bzw. Aufreinigung geschieht aus dem Eluat nach Abtrennung vom Gel in oben beschriebener Weise.

Schließlich kann die Reduktion des betreffenden Ketostatins nach dem erfindungsgemäßen Verfahren auch mit einem zellfreien Extrakt durchgeführt werden. Hierzu werden die Zellen in bekannter Weise homogenisiert bzw. aufgeschlossen. Dem daraus gewonnenen zellfreien Extrakt, der die wasserstoffübertragenden Enzyme und Co-Faktoren in gelöster Form enthält, werden Glucose und Ketostatin in den angegebenen Konzentrationen zugesetzt, und die Reduktion wird zwischen 25 und 30 °C mehrere Stunden vollzogen. Das weitere Prozedere wird, wie beschrieben, durchgeführt.

Die Reduktion von Ketonen der Formel I zu den sekundären Alkoholen der Formel II mit Hilfe von reduzierenden Mikroorganismen, insbesondere Hefen, liefert folgende vorteilhafte Ergebnisse: Die Ausbeute an Alkohol variiert zwischen 50 und 98 %, bezogen auf das Edukt, je nach verwendetem Mikroorganismus, eingesetztem Ketostatin und Ausführungsform. Die besten Ausbeuten liefern nach dem Extraktionsverfahren Ketostatine der Formel I mit R¹ = Benzyl und R² = Methyl, gefolgt von Verbindungen mit R¹ = 4-Methyl-cyclohexylmethyl und R¹ = 4-tert.-Butyl-cyclohexylmethyl und jeweils R² = Methyl. Die Ausbeuten für die nicht geschützten Verbindungen sind ebenfalls ausgezeichnet. Bezogen auf die gebildeten Alkohole liegen 90-99 %, vorzugsweise 95-98 % in der biologisch aktiven S-Konfiguration (am OH-Gruppe tragenden C-Atom) vor, der Rest weist die R-Konfiguration auf. Von den eingesetzten Hefen produziert die Gattung Hansenula und darin insbesondere die Arten Hansenula ciferrii, Hansenula saturnus und Hansenula anomala die größten Ausbeuten insgesamt und an 3S-Enantiomeren. Die Art der Ausführungsform spielt, bezogen auf Ausbeute und Enantiomerenverhältnis, in der Regel nur eine untergeordnete Rolle.

Die nach dem erfindungsgemäßen Verfahren hergestellten sekundären Alkohole der Formel II stellen wichtige Zwischenprodukte in der Synthese von Renin-Hemmern dar, die in der Pharmakologie als effektive und wertvolle Blutdrucksenker eingesetzt werden können. Bislang weisen nahezu alle bekannten Renin-Inhibitoren das StatinStrukturelement -NH- 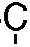 H-CHOH-CH₂-CO- auf.

### Beispiel 1:

In einem 500-ml-Erlenmeyerkolben werden 2 g Hefezellen der Art Hansenula ciferrii (DSM 70780) in 100 ml 20%iger Glucoselösung suspendiert. Nach einsetzender Gärung werden dem Ansatz 200 mg 5-Phenyl-4(S)-BOC-amino-3-oxo-valeriansäuremethylester, gelöst in 5 ml Ethanol, zugesetzt. Die Inkubation erfolgt bei 28 °C unter ständigem Rühren. Nach 48 h werden weitere 10 g Glucose zugesetzt und die Inkubation für weitere 24 Stunden fortgesetzt. Die Inkubation wird durch Zugabe von Ethylacetat gestoppt und die Mischung eine weitere Stunde gerührt. Nach Trennung der Phasen wird die organische Phase mit Natriumsulfat getrocknet und eingeengt. Der Rückstand wird in Cyclohexan/tert.-Butylmethylether aufgenommen und an Kieselgel chromatographiert. Eluent: Cyclohexan/tert.-Butylmethylether (1:1). Zum Nachweis der Umsetzung und Bestimmung des Enantiomerenverhältnisses wird eine Probe von Edukt und Produkt mittels HPLC wie folgt chromatographiert. Säule: RP 8, Fließmittel: CH₃CN/0,1 M NaH₂PO₄ (1:1), Detektion bei 220 nm. Man erhält 5-Phenyl-4(S)-BOC-amino-3(S)-hydroxy-valeriansäuremethylester; Enantiomerenreinheit: 97 %.

### Beispiel 2:

100 g kultivierte Hefezellen der Art Hansenula saturnus (DSM 70278) werden in 10 l 10%iger Glucoselösung suspendiert und 10 g 5-Phenyl-4(S)-BOC-amino-3-oxo-valeriansäuremethylester, gelöst in 100 ml Ethanol, zugesetzt. Die Inkubation erfolgt unter leichter Belüftung und ständigem Rühren bei 28 °C. Die Inkubation wird nach 72 Stunden durch Zugabe von insgesamt 5 l Ethylacetat gestoppt. Die organische Phase wird über Kieselgur abgesaugt, mit Natriumsulfat getrocknet und umkristallisiert. Man erhält 5-Phenyl-4(S)-BOC-amino-3(S)-hydroxyvaleriansäuremethylester; Enantiomerenreinheit: 95 %.

### Beispiel 3:

150 mg 5-(4-Methylcyclohexyl)-4(S)-BOC-amino-3-oxo-valeriansäuremethylester (gelöst in 5 ml Ethanol) werden mit 100 ml 1,5 g Hefezellen der Art Hansenula ciferrii enthaltender 15%iger Glucoselösung versetzt und analog Beispiel 1 umgesetzt. Man erhält 5-(4-Methylcyclohexyl)-4(S)-BOC-amino-3(S)-hydroxy-valeriansäuremethylester; Enantiomerenreinheit: 80 %.

### Beispiel 4:

Analog Beispiel 1 werden 200 mg 5-(4-tert.-Butylcyclohexyl)-4(S)-BOC-amino-3-oxo-valeriansäure mit 2 g käuflicher Bäckerhefe (Saccharomyces cerevisiae) umgesetzt und aufgearbeitet. Man erhält 5-(4-tert.-Butylcyclohexyl)-4(S)-BOC-amino-3S-hydroxy-valeriansäure; Enantiomerenreinheit: 64 %.

### Beispiel 5:

10 g kultivierte Hefezellen der Art Hansenula saturnus werden in 1 l 15%iger Glucoselösung suspendiert und 1 g 5-Phenyl-4(S)-amino-3-oxo-valeriansäuremethylester (gelöst in 20 ml Ethanol) zugesetzt. Die Inkubationsbedingungen entsprechen denen in Beispiel 1. Nach Beendigung der Inkubation werden die Hefezellen abzentrifugiert und mit Wasser nachgewaschen. Die vereinigten wäßrigen Überstände werden anschließend gefriergetrocknet. Man erhält 5-Phenyl-4(S)-amino-3(S)-hydroxy-valeriansäuremethylester; Enantiomerenreinheit: 90 %.

### Beispiel 6:

1 g Zellen von Hansenula ciferrii werden in 1 ml 0,9%iger NaCl-Lösung suspendiert und die Suspension auf 4 °C gekühlt. Diese Hefezellen werden zu gleichen Teilen mit einer Lösung von 750 mg Acrylamid und 40 mg N,N′-Methylenbisacrylamid in 2,4 ml H₂O gemischt. Die Polymerisation wird durch Zugabe von 0,1 ml einer 25%igen β-Dimethylaminopropionitril- und 0,5 ml einer 1%igen Kaliumperoxidisulfat-Lösung gestartet. Nach erfolgter Polymerisation wird das Gel zerkleinert und anstelle "freier" Hefezellen analog Beispiel 1 zur Reduktion von 100 mg 5-Phenyl-4(S)-BOC-amino-3-oxo-valeriansäuremethylester eingesetzt. Man erhält 5-Phenyl-4(S)-BOC-amino-3(S)-hydroxy-valeriansäuremethylester; Enantiomerenreinheit: 97 %.

### Beispiel 7:

2 g käufliche Bäckerhefe werden mit 2 ml 0,1 N Tris/HCl-Puffer, pH 7,5 und 4 g Glasperlen (Durchmesser: 0,45-0,50 mm) versetzt und im Zellhomogenisator bei ca. 4000 U/min bei 0 bis 4 °C aufgeschlossen. Glasperlen und Zelltrümmer werden bei ca. 6000 x g 20 Minuten abzentrifugiert. Dem nun zellfreien Extrakt werden je 20 % Glucose (w/v) und 0,1 % 5-Phenyl-4(S)-amino-3-oxo-valeriansäuremethylester zugesetzt. Unter leichtem Rühren wird der Ansatz bei 28 °C mehrere Stunden inkubiert. Nach abgeschlossener Inkubation werden mit Aceton die löslichen Proteine ausgefällt; anschließend wird mehrere Male mit Ethylacetat ausgeschüttelt. Aus der wäßrigen Phase wird denaturiertes Protein abzentrifugiert und mit Wasser gewaschen. Die vereinigten wäßrigen Extrakte werden gefriergetrocknet. Man erhält 5-Phenyl-4(S)-amino-3(S)-hydroxy-valeriansäuremethylester; Enantiomerenreinheit: 92 %.

## Patentansprüche

1. Verfahren zur Herstellung von am β-C-Atom S-konfigurierten sekundären Alkoholen der Formel II
R³-HN-CHR¹-CHOH-CH₂-COOR² II
worin
R¹ unsubstituiertes oder durch Halogen, Alkyl oder Alkoxy einfach substituiertes Phenyl, Benzyl, Phenylethyl, Cyclohexyl, Cyclohexylmethyl oder -ethyl, Naphtylmethyl oder -ethyl oder Alkyl mit 1 - 8 C-Atomen,
R² H oder Alkyl mit 1-5 C-Atomen und
R³ H, Benzyl, Triphenylmethyl, Benzyloxycarbonyl, tert.-Butyloxycarbonyl oder 9-Fluorenylmethoxycarbonyl
bedeuten,
dadurch gekennzeichnet, daß man die β-Ketostatinderivate der Formel I
R³-HN-CHR¹-CO-CH₂-COOR² I
worin R¹, R² und R³ die angegebenen Bedeutungen haben enantioselektiv mit Hilfe von Mikroorganismen reduziert.

2. Verfahren nach Anspruch 1, dadurch gekennzeichnet, daß Verbindungen der Formel I eingesetzt werden, worin
R¹ Benzyl, Cyclohexylmethyl, 4-Methylcyclohexylmethyl, 4-tert.-Butylcyclohexylmethyl, Phenyl, 2-Phenylethyl, 2-Cyclohexylethyl, Methyl, Ethyl, Propyl, Isopropyl, Butyl, Isobutyl, sec.-Butyl, Pentyl oder 2- oder 3-Methylbutyl,
R² Methyl, Ethyl oder Isopropyl und
R³ H, Benzyloxycarbonyl oder ter.-Butyloxycarbonyl
bedeuten.

3. Verfahren nach Anspruch 1 oder 2, dadurch gekennzeichnet, daß man Dehydrogenasen enthaltende Mikroorganismen, insbesondere Hefen einsetzt.

4. Verfahren nach Anspruch 3, dadurch gekennzeichnet, daß man Hefen der Gattungen Saccharomyces, Candida, Hansenula, Pullularia, Torulopsis oder Williopsis, insbesondere der Arten Hansenula ciferrii, Hansenula saturnus oder Hansenula anomala einsetzt.

5. Verfahren nach einem der Ansprüche 1 bis 4, dadurch gekennzeichnet, daß die Ketostatinkonzentration zwischen 0.05 und 0.5% und die Inkubationsdauer zwischen 24 und 84 Stunden liegt.

6. Verfahren nach Anspruch 5, dadurch gekennzeichnet, daß man Hefen gemäß Anspruch 4 in einer Konzentration zwischen 0.5 und 5%, insbesondere zwischen 1 und 2% einsetzt, und die Inkubationsdauer Zwischen 48 und 72 Stunden liegt.

7. Verfahren nach einem der Ansprüche 1 bis 6, dadurch gekennzeichnet, daß man die Reduktion in Anwesenheit von Ethanol in einer Konzentration zwischen 3 - 10 % durchführt.

8. Verfahren nach einem der Ansprüche 1 bis 7, dadurch gekennzeichnet, daß man durch eine Trägermatrix immobilisierte Zellen einsetzt.

9. Verfahren zur Herstellung von am β-C-Atom S-konfigurierten sekundären Alkoholen der Formel II
R³-HN-CHR¹-CHOH-CH₂-COOR² II
worin
R¹ A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen oder Bicycloalkyl mit 7-14 C-Atomen oder Bicycloalkylalkyl mit 8-18 C-Atomen,
R² H oder Alkyl mit 1-5 C-Atomen
R³ H, Benzyl, Triphenylmethyl, Benzyloxycarbonyl, tert.-Butyloxycarbonyl oder 9-Fluorenylmethoxycarbonyl,
Ar unsubstituiertes oder ein- oder mehrfach durch A, AO, Hal, CF₃, HO, Hydroxyalkyl mit 1-8 C-Atomen, H₂N und/oder Aminoalkyl mit 1-8 C-Atomen substituiertes Phenyl oder unsubstituiertes Naphthyl,
Het einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, CF₃, HO, O₂N, Carbonylsauerstoff, H₂N, HAN, A₂N, AcNH, AS, ASO, ASO₂, AOOC, CN, H₂NCO, HOOC, H₂NSO₂, ASO₂NH, Ar, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,
Hal F, Cl, Br oder J,
Ac A-CO-, Ar-CO- oder A-NH-CO- und
A Alkyl mit 1-8 C-Atomen bedeuten,
dadurch gekennzeichnet, daß man die β-Ketostatinderivate der Formel I
R³-HN-CHR¹-CO-CH₂-COOR² I
worin R¹, R² und R³ die angegebenen Bedeutungen haben, enantioselektiv mit Hilfe von Hefen der Gattungen Saccharomyces, Candida, Hansenula, Pullularia, Torulopsis oder Williopsis, insbesondere der Arten Hansenula ciferrii, Hansenula saturnus oder Hansenula anomala, reduziert, wobei die Ketostatinkonzentration zwischen 0.05 und 0.5 %, die Hefekonzentration zwischen 0.5 und 5 % und die Inkubationsdauer zwischen 24 und 84 Stunden liegen, und die Reduktion in Anwesenheit von Ethanol in einer Konzentration von 3 - 10% durchgeführt wird.

## Claims

1. Process for the preparation of secondary alcohols having the S configuration on the β-C atom of the formula II
R³-HN-CHR¹-CHOH-CH₂-COOR² II
in which
R¹ is unsubstituted or halogen-, alkyl- or alkoxy-monosubstituted phenyl, benzyl, phenylethyl, cyclohexyl, cyclohexylmethyl or -ethyl, naphthylmethyl or -ethyl or alkyl having 1-8 C atoms,
R² is H or alkyl having 1-5 C atoms and
R³ is H, benzyl, triphenylmethyl, benzyloxycarbonyl, tert-butyloxycarbonyl or 9-fluorenylmethoxycarbonyl,
characterised in that the β-ketostatin derivatives of the formula I
R³-HN-CHR¹-CO-CH₂-COOR² I
in which R¹, R² and R³ have the meanings given are reduced enantioselectively by means of microorganisms.

2. Process according to Claim 1, characterised in that compounds of the formula I are used in which
R¹ is benzyl, cyclohexylmethyl, 4-methylcyclohexylmethyl, 4-tert.-butylcyclohexylmethyl, phenyl, 2-phenylethyl, 2-cyclohexylethyl, methyl, ethyl, propyl, isopropyl, butyl, isobutyl, sec.-butyl, pentyl or 2- or 3-methylbutyl,
R² is methyl, ethyl or isopropyl and
R³ is H, benzyloxycarbonyl or ter.-butyloxycarbonyl.

3. Process according to Claim 1 or 2, characterised in that microorganisms containing dehydrogenases, in particular yeasts, are used.

4. Process according to Claim 3, characterised in that yeasts of the genera Saccharomyces, Candida, Hansenula, Pullularia, Torulopsis or Williopsis, in particular of the genera Hansenula ciferrii, Hansenula saturnus or Hansenula anomala, are used.

5. Process according to one of Claims 1 to 4, characterised in that the ketostatin concentration is between 0.05 and 0.5% and the incubation time is between 24 and 84 hours.

6. Process according to Claim 5, characterised in that yeasts according to Claim 4 are used in a concentration of between 0.5 and 5%, in particular of between 1 and 2%, and the incubation time is between 48 and 72 hours.

7. Process according to one of Claims 1 to 6, characterised in that the reduction is carried out in the presence of ethanol in a concentration of between 3 - 10%.

8. Process according to one of Claims 1 to 7, characterised in that cells immobilised by a support matrix are used.

9. Process for the preparation of secondary alcohols having the S configuration on the β-C atom of the formula II
R³-HN-CHR¹-CHOH-CH₂-COOR² II
in which
R¹ is A, Ar, Ar-alkyl, Het, Het-alkyl, cycloalkyl having 3-7 C atoms which is unsubstituted or mono- or polysubstituted by A, AO and/or Hal, cycloalkylalkyl having 4-11 C atoms or bicycloalkyl having 7-14 C atoms or bicycloalkylalkyl having 8-18 C atoms,
R² is H or alkyl having 1-5 C atoms,
R³ is H, benzyl, triphenylmethyl, benzyloxycarbonyl, tert.-butyloxycarbonyl or 9-fluorenylmethoxycarbonyl,
Ar is phenyl which is unsubstituted or mono- or polysubstituted by A, AO, Hal, CF₃, HO, hydroxyalkyl having 1-8 C atoms, H₂N and/or aminoalkyl having 1-8 C atoms or is unsubstituted naphthyl,
Het is a saturated or unsaturated 5- or 6-membered heterocyclic radical having 1-4 N, O and/or S atoms which can be fused with a benzene ring and/or mono- or polysubstituted by A, AO, Hal, CF₃, HO, O₂N, carbonyl oxygen, H₂N, HAN, A₂N, AcNH, AS, ASO, ASO₂, AOOC, CN, H₂NCO, HOOC, H₂NSO₂, ASO₂NH, Ar, Ar-alkenyl, hydroxyalkyl and/or aminoalkyl each having 1-8 C atoms and/or whose N and/or S heteroatoms can also be oxidised,
Hal is F, Cl, Br or I,
Ac is A-CO-, Ar-CO- or A-NH-CO- and
A is alkyl having 1-8 C atoms,
characterised in that the β-ketostatin derivatives of the formula I
R³-HN-CHR¹-CO-CH₂-COOR² I
in which R¹, R² and R³ have the meanings given, are reduced enantioselectively by means of yeasts of the genera Saccharomyces, Candida, Hansenula, Pullularia, Torulopsis or Williopsis, in particular of the genera Hansenula ciferrii, Hansenula saturnus or Hansenula anomala, the ketostatin concentration being between 0.05 and 0.5%, the yeast concentration between 0.5 and 5%, and the incubation time between 24 and 84 hours, and the reduction being carried out in the presence of ethanol in a concentration of 3 - 10%.

## Revendications

1. Procédé de préparation d'alcools secondaires en configuration S sur l'atome de carbone bêta, de formule II
R³NH-CHR¹-CHOH-CH₂-COOR² II,
dans laquelle
R¹ représente un groupe phényle, benzyle, phényléthyle, cyclohexyle, cyclohexylméthyle ou cyclohexyléthyle, naphtylméthyle ou naphtyléthyle ou alkyle en c 1-C 8 non substitué ou portant un substituant halogéno, alkyle ou alcoxy,
R² représente H ou un groupe alkyle en C 1-C 5, et
R³ représente H, un groupe benzyle, triphénylméthyle, benzyloxycarbonyle, tert-butyloxycarbonyle ou 9-fluorénylméthoxycarbonyle,
caractérisé en ce que l'on soumet les dérivés de bêta-cétostatines de formule I
R³-HN-CHR¹-CO-CH₂-COOR² I,
dans laquelle R¹, R² et R³ ont les significations indiquées ci-dessus, à réduction énantiosélective à l'aide de microorganismes.

2. Procédé selon la revendication 1, caractérisé en ce que l'on met en oeuvre des composés de formule I dans laquelle
R¹ représente un groupe benzyle, cyclohexylméthyle, 4-méthylcyclohexylméthyle, 4-tert-butylcyclohexylméthyle, phényle, 2-phényléthyle, 2-cyclohexyléthyle, méthyle, éthyle, propyle, isopropyle, butyle, isobutyle, sec-butyle, pentyle ou 2- ou 3-méthylbutyle,
R² représente un groupe méthyle, éthyle ou isopropyle, et
R³ représente H, un groupe benzyloxycarbonyle ou tert-butyloxycarbonyle.

3. Procédé selon la revendication 1 ou 2, caractérisé en ce que l'on utilise des microorganismes contenant des déshydrogénases, en particulier des levures.

4. Procédé selon la revendication 3, caractérisé en ce que l'on utilise des levures des genres Saccharomyces, Candida, Hansenula, Pullularia, Torulopsis ou Williopsis, en particulier des espèces Hansenula ciferrii, Hansenula saturnus ou Hansenula anomala.

5. Procédé selon l'une des revendications 1 à 4, caractérisé en ce que la concentration en cétostatine est de 0,05 à 0,5% et la durée d'incubation de 24 h à 84 h.

6. Procédé selon la revendication 5, caractérisé en ce que l'on utilise des levures selon revendication 4 à une concentration de 0,5 à 5%, plus spécialement de 1 à 2%, avec des durées d'incubation de 48 à 72 h.

7. Procédé selon une des revendications 1 à 6, caractérisé en ce que l'on procède à la réduction en présence d'éthanol à une concentration de 3 à 10%.

8. Procédé selon une des revendications 1 à 7, caractérisé en ce que l'on utilise des cellules immobilisées sur un support.

9. Procédé de préparation d'alcools secondaires en configuration S sur l'atome de carbone bêta, de formule II
R³-HN-CHR¹-CHOH-CH₂-COOR² II
dans laquelle
R¹ représente A, Ar, Ar-alkyle, Het, Het-alkyle, cycloalkyle en C 3-C 7 non substitué ou portant un ou plusieurs substituants A, AO et/ou Hal, un groupe cycloalkylalkyle en C 4-C 11 ou bicycloalkyle en C 7-C 14 ou bicycloalkylalkyle en C 8-C 18,
R² représente H ou un groupe alkyle en C 1-C 5,
R³ représente H, un groupe benzyle, triphénylméthyle, benzyloxycarbonyle, tert-butyloxycarbonyle ou 9-fluorénylméthoxycarbonyle,
Ar représente un groupe phényle non substitué ou portant un ou plusieurs substituants A, AO, Hal, CF₃, HO, hydroxyalkyle en C 1-C 8, H₂N et/ou aminoalkyle en C 1-C 8, ou un groupe naphtyle non substitué,
Het représente un groupe hétérocyclique saturé ou insaturé à 5 ou 6 chaînons contenant 1 à 4 atomes de N, de O et/ou de S, qui peut être condensé avec un noyau benzénique et/ou porter un ou plusieurs substituants A, AO, Hal, CF₃, HO, O₂N, oxygène de carbonyle, H₂N, HAN, A₂N, AcNH, AS, ASO, ASO₂, AOOC, CN, H₂NCO, HOOC, H₂NSO₂, ASO₂NH, Ar, Ar-alcényle, hydroxyalkyle et/ou aminoalkyle contenant chacun 1 à 8 atomes de carbone et/ou dont les hétéroatomes de N et/ou de S peuvent également être oxydés,
Hal représente F, Cl, Br ou I,
Ac représente A-CO-, Ar-CO- ou A-NH-CO-, et
A représente un groupe alkyle en C 1-C 8,
caractérisé en ce que l'on soumet les dérivés de bêta-cétostatines de formule I
R³-HN-CHR¹-CO-CH₂-COOR² I
dans laquelle R¹, R² et R³ ont les significations indiquées ci-dessus, à réduction énantiosélective à l'aide de levure des genres Saccharomyces, Candida, Hansenula, Pullularia, Torulopsis ou Williopsis, en particulier des espèces Hansenula ciferrii, Hansenula saturnus ou Hansenula anomala, à une concentration en cétostatine de 0,05 à 0,5%, une concentration en levure de 0,5 à 5% et une durée d'incubation de 24 à 84 h, la réduction étant effectuée en présence d'éthanol à une concentration de 3 à 10%.
